# EUROPÄISCHE PATENTSCHRIFT

(11) **EP 2 612 300 B1**
(45) Veröffentlichungstag und Bekanntmachung des Hinweises auf die Patenterteilung: **25.02.2015**
(21) Anmeldenummer: 11758426.8
(22) Anmeldetag: 01.09.2011
(51) Int. Cl.: G06T 15/08, G06T 11/00

(54) **VERFAHREN ZUR ERSTELLUNG EINER AUFNAHME AUS EINEM 3D-VOLUMEN**
METHOD FOR CREATING AN IMAGE FROM A 3D VOLUME
PROCÉDÉ POUR GÉNÉRER UNE IMAGE À PARTIR D'UN VOLUME 3D

(30) Priorität: 01.09.2010 DE 102010040096
(43) Veröffentlichungstag der Anmeldung: 10.07.2013
(73) Patentinhaber: Sirona Dental Systems GmbH, 64625 Bensheim (DE)
(72) Erfinder: ULRICI, Johannes, 64293 Darmstadt (DE)
(74) Vertreter: Sommer, Peter
(86) Internationale Anmeldenummer: PCT/EP2011/065065
(87) Internationale Veröffentlichungsnummer: WO 2012/028670

(56) Entgegenhaltungen:
- DE-A1-102008 008 733
- DE-A1-102008 021 926
- C. JACKOWSKI ET AL: "Ultra-high-resolution dual-source CT for forensic dental visualization-discrimination of ceramic and composite fillings", INTERNATIONAL JOURNAL OF LEGAL MEDICINE, Bd. 122, Nr. 4, 1. Juli 2008 (2008-07-01), Seiten 301-307, XP55016112, ISSN: 0937-9827, DOI: 10.1007/s00414-008-0224-8

## Beschreibung

### Technisches Gebiet

Die Erfindung betrifft ein Verfahren zur Erstellung einer Aufnahme aus einem 3D-Volumen mit volumetrischen Bilddaten von einem Kieferbogen und von Zähnen.

### Stand der Technik

Aus dem Stand der Technik sind mehrere Verfahren zur Erstellung einer virtuellen dreidimensionalen Röntgenaufnahme bekannt. Bei der so genannten digitalen Volumentomographie (DVT) werden ein Strahler und ein Sensor, die durch eine Achse miteinander verbunden sind, in einer definierten Ebene um den Kopf des Patienten bewegt. Aus verschiedenen Positionen in dieser Umlaufbahn werden Projektionsaufnahmen erzeugt und im nächsten Schritt aus diesen Projektionsaufnahmen anschließend ein dreidimensionales Volumen berechnet. Das 3D-Volumen kann dann in einer Benutzersoftware auf einem Bildschirm dargestellt werden. Bei einer so genannten Computertomografie (CT) beruht die Bildgebung auf einer kontinuierlichen Aufnahme der Projektion aus unterschiedlichen Richtungen. In der Regel setzen sich die berechneten 3D-Rekonstruktionen aus Einzelschnitten nach dem Schnittbildverfahren zusammen, die quer durch das Objekt verlaufen. Auf diese Weise kann für jedes Volumenelement des Objekts, so genannte Voxel, der Röntgenabsorptionswert ermittelt werden.

In der DE 101 08 295 A1 ist ein Verfahren und eine Anordnung zur Identifikation von Objekten, insbesondere von Zähnen, offenbart, basierend auf einer digitalisierten Röntgenaufnahme, bei unter Verwendung von Bildverarbeitungsalgorithmen durch Segmentierung und/oder Kantendetektion im Objekt enthaltene Bereiche eingegrenzt und mit Parametern des Röntgengeräts sowie ggf. der Patienten zu spezifischen Parametern rechnerisch verknüpft werden. Darüber hinaus ist ein Verfahren offenbart, bei dem die Bestimmung der Objekte manuell oder automatisch erfolgt, wobei in einem Schritt das Objekt ausgewählt wird, für das weitere Informationen gespeichert, abgerufen oder gelöscht werden sollen, und in einem weiteren Schritt eine Referenz in Relation zum Objekt abgelegt wird, um anhand dieser Referenz die Informationen zu bestimmen, die dargestellt werden.

Auf die Bilddaten der digitalisierten Röntgenaufnahme werden computergestützt die Verfahren der Kantenfindung und der Segmentierung angewendet und die ermittelten Kanten und Segmente mittels der so genannten Cluster-Bildung gruppiert.

Das Patent US 5,179,579 offenbart ein Verfahren zur Darstellung von intraoralen Röntgenaufnahmen. Die intraoralen Röntgenaufnahmen werden erfasst, digitalisiert und zusammen mit einem Symbol eines Teils der Anatomie, von der die Röntgenaufnahme aufgenommen wird, angezeigt. Die Aufnahmen der anatomischen Bereiche werden mit dem jeweiligen Symbol nach ihrer normalen anatomischen Anordnung auf einem Bildschirm angezeigt. Das Symbol wird durch den Benutzer verwendet, um die jeweilige Röntgenaufnahme eines Bereiches der Anatomie auszuwählen. Als Symbol kann auch ein verkleinertes Bild des Gebisses, einer Zahnreihe oder der einzelnen Zähne verwendet werden.

In der US 6,190,042 B1 ist eine Vorrichtung für verbesserte intraorale Röntgenaufnahmen offenbart. Die Vorrichtung beinhaltet einen Aufbissblock, eine Führungsstange, einen Zielring und einen zusätzlichen Ring. Der Aufbissblock enthält einen Filmhalter, der senkrecht zu der Oberseite des Aufbissblocks angeordnet ist. Die Führungsstange ist dabei mit dem Aufbissblock verbunden. Mittels der Vorrichtung wird ein vorgegebener Abstand zwischen dem Film im Filmhalter und dem äußeren Ring gewährleistet.

In einem Artikel von C. Jackowski et al., "Ultra-high-resolution dual-source CT for forensic dental visualizationdiscrimination of ceramic and composite fillings", INTERNATIONAL JOURNAL OF LEGAL MEDICINE, S. 301-307, 2008, wird ein Verfahren gemäss des Oberbegriffes von Anspruch 1 beschrieben.

Ein Nachteil der genannten Vorrichtungen und Verfahren aus dem Stand der Technik ist, dass zur Erstellung der intraoralen Aufnahme eine aufwändige Vorrichtung im Mund des Patienten positioniert und fixiert werden muss, wobei diese Vorrichtung zur Fixierung meist einen Aufbissblock aufweist, und als Aufnahmemittel ein Röntgenfilm oder ein digitaler Röntgensensor an dieser Vorrichtung im Mundraum des Patienten angeordnet ist. Die Positionierung dieser Vorrichtung wird dadurch erschwert, dass der Patient beim Aufbeißen die relative Position der Vorrichtung zum Oberkiefer verändert und dass der Abstand des Aufnahmeelements von den Zähnen unbekannt ist.

Bei einer Serie von mehreren intraoralen Aufnahmen kann es vorkommen, dass durch die ungenaue Positionierung Lücken zwischen den einzelnen Aufnahmen entstehen und dadurch der Mundraum des Patienten nicht vollständig aufgenommen wird. Darüber hinaus ist das Aufnahmevolumen der intraoralen Aufnahmen bezogen auf die Zähne in der Aufnahmerichtung nicht bekannt.

In der DE 10 2008 008 733 A1 ist ein Verfahren zur Erstellung einer Schichtaufnahme offenbart, wobei aus einem digitalen 3D-Volumen, das Röntgenstrahlenabsorptionswerte aufweist, insbesondere eine dentale Röntgen-Panoramaschichtaufnahme erzeugt wird, wobei das 3D-Volumen als aufzunehmendes Objekt mit einer virtuellen Röntgenquelle virtuell durchstrahlt und das virtuelle entstehende Bild mit einem virtuellen Detektor aufgenommen wird. Die virtuelle Röntgenquelle und der virtuelle Detektor werden unter Ausbildung einer scharfen Schicht mit einem Verwischungsanteil an dem aufzunehmenden Objekt virtuell vorbeibewegt. Die Breite des virtuellen Detektors, die Auffächerung des virtuellen Strahlenfächers und die simulierte Umlaufgeschwindigkeit der virtuellen Röntgenquelle und des virtuellen Detektors ist veränderbar, um die Dicke sowie die Position der aufgenommen scharfen Schicht zu beeinflussen. In einer Ausführungsform wird aus dem 3D-Volumen ein festgelegter Teilbereich ausgewählt, dessen Ausdehnung der scharfen Schicht entspricht, wobei bei einer virtuellen Durchstrahlung des Teilbereichs senkrecht zum Verlauf des Teilbereichs eine Panoramaaufnahme erzeugt wird.

Ein Nachteil dieses Verfahrens besteht darin, dass die virtuell erzeugte Panoramaaufnahme im Gegensatz zu intraoralen Projektionsaufnahmen ein verzerrtes Bild des gesamten Kieferbogens darstellt und dadurch die Diagnose bestimmter Befunde erschwert wird.

Die Aufgabe dieser Erfindung besteht daher darin, eine zweidimensionale Projektionsaufnahme bei einem definierten Aufnahmevolumen zu erzeugen.

### Darstellung der Erfindung

Ein Gegenstand der vorliegenden Erfindung ist ein Verfahren zur Erstellung einer virtuellen dentalen Aufnahme aus einem 3D-Volumen mit volumetrischen Bilddaten. Aus dem 3D-Volumen wird zunächst ein Teilvolumen festgelegt. Für dieses Teilvolumen wird eine virtuelle Projektionsaufnahme aus einer bestimmten Durchstrahlrichtung durch computergestützte Verrechnung der volumetrischen Bilddaten in dieser Durchstrahlrichtung erzeugt.

Das 3D-Volumen kann eine dreidimensionale Röntgenaufnahme sein, die mittels eines beliebigen dreidimensionalen Röntgenaufnahmeverfahrens, wie DVT oder CT, erzeugt worden ist und wobei die volumetrischen Bilddaten aus Voxeln mit Röntgenabsorptionswerten bestehen können. Das 3D-Volumen kann auch mittels anderer bildgebender Verfahren, wie MRT, aufgenommen sein. Das 3D-Volumen kann vom gesamten Kieferbogen mit Zähnen oder von einem Teil davon vorliegen. Die volumetrischen Bilddaten des 3D-Volumens können aus Voxeln, Vektorelementen oder aus einer Kombination von Voxeln und Vektorelementen aufgebaut sein. Die volumetrischen Bilddaten können auch aus Punktwolken bestehen, die Vektorelemente enthalten. Aus diesem 3D-Volumen wird ein bestimmtes Teilvolumen mit einer beliebigen Grundfläche und einer bestimmten Tiefe in der Durchstrahlrichtung ausgewählt. In einem weiteren Schritt wird für dieses ausgewählte Teilvolumen eine virtuelle Projektionsaufnahme durch Verrechnung der volumetrischen Bilddaten, wie Voxel oder Vektorelemente, in Durchstrahlrichtung erzeugt. Dadurch entsteht eine virtuelle Projektionsaufnahme des ausgewählten Teilvolumens, die einer intraoralen Röntgenaufnahme eines bestimmten Bereichs des Kieferbogens entspricht. Die Verrechnung der Volumetrischen Bilddaten in Durchstrahlrichtung kann beispielsweise durch Summation der einzelnen hintereinander angeordneten volumetrischen Bilddaten, wie Voxel oder Vektorelemente, in Durchstrahlrichtung erfolgen. Die Verrechnung der volumetrischen Bilddaten kann auch durch Integration nach der Anwendung einer bestimmten Funktion auf die in Durchstrahlrichtung angeordneten Voxel erfolgen.

Vor der Verrechnung der volumetrischen Bilddaten kann eine bestimmte Funktion auf die volumetrischen Bilddaten angewendet werden, um die volumetrischen Bilddaten in Durchstrahlrichtung so zu gewichten, dass bestimmte für die Auswertung wesentliche Bereiche, wie beispielsweise mit Karies befallene Bereiche, hervorgehoben werden und andere für die Auswertung unwesentliche Bereiche, wie beispielsweise eine Füllung, weniger gewichtet werden. Eine solche Funktion kann beispielsweise auf die Grauwerte der einzelnen Elemente der volumetrischen Bilddaten angewendet werden.

Das 3D-Volumen kann aus Voxeln bestehen, die Volumenelemente des aufgenommenen Objekts repräsentieren. Als 3D-Volumen kann dabei jede beliebige dreidimensionale dentale Aufnahme verwendet werden, wie eine dreidimensionale Röntgenaufnahme, nämlich eine DVT-Aufnahme oder eine CT-Aufnahme mit Voxeln, die Röntgenabsorptionswerte in Form von Grauwerten enthalten, oder eine MRT-Aufnahme mit Voxeln, die Volumenelemente des aufgenommenen Gewebes repräsentieren. Das 3D-Volumen kann auch eine dreidimensionale Ultraschallaufnahme sein.

Bei der Erzeugung der Projektionsaufnahme können die volumetrischen Bilddaten durch Summation in der Durchstrahlrichtung erfolgen, wobei die virtuelle Durchstrahlrichtung über die gesamte Grundfläche parallel ist oder in Form eines konischen Fächers als Verlängerung von virtuellen Röntgenstrahlen einer virtuellen Röntgenquelle gebildet ist. Die Grundfläche kann dabei beliebig gestaltet sein, beispielsweise in Form eines Kreises, eines Rechtecks oder eines festgelegten Umrisses des aufzunehmenden Objekts.

Das Teilvolumen kann auch eine andere beliebige geometrische Grundform, wie einen Tetraeder, oder die Form eines segmentierten Teilvolumens haben.

Eine optimale Projektionsaufnahme wird mit einer Grundfläche in Form eines Rechtecks in einer parallelen Durchstrahlrichtung erzeugt, so dass das ausgewählte Teilvolumen die Form eines Quaders hat.

Die Projektionsaufnahme sollte in ihren Abmessungen der Größe einer herkömmlichen Intraoralaufnahme mit Zahnfilmen entsprechen, nämlich mit einer Breite zwischen 15 mm und 30 mm und einer Höhe zwischen 25 mm und 40 mm.

Ein Vorteil ist, dass zur Erzeugung der Projektionsaufnahmen der Patient nicht nochmals bestrahlt werden muss. Die Projektionsaufnahmen, wie intraorale Röntgenaufnahmen bestimmter Bereiche, können aus den bereits vorhandenen dreidimensionalen Bilddaten des 3D-Volumens erzeugt werden.

Ein weiterer Vorteil ist, dass das Teilvolumen computergestützt durch den Benutzer genau festgelegt werden kann und beispielsweise intraorale Röntgenaufnahmen einzelner Zähne erzeugt werden können.

Ein weiterer Vorteil liegt darin, dass das Teilvolumen so gewählt werden kann, dass ein Gebiet des 3D-Volumens von Interesse, wie eine Füllung, virtuell ausgeschnitten wird, um eine Projektionsaufnahme ohne den ausgeschnittenen Bereich zu berechnen. Dadurch kann beispielsweise eine Sekundärkaries, die unter der Füllung liegt, diagnostiziert werden. Dieses Herausschneiden spezieller Strukturen lässt sich bei Füllungen, Dentinschichten, Zahnschmelzstrukturen etc. durchführen, die nach einer entsprechenden Segmentierung virtuell ausgeschnitten werden können.

Vorteilhafterweise kann das 3D-Volumen mit volumetrischen Bilddaten eine dreidimensionale Röntgenaufnahme mit Röntgenabsorptionswerten sein.

Dadurch entspricht die virtuelle Projektionsaufnahme einer zweidimensionalen Röntgenaufnahme in einer bestimmten Durchstrahlrichtung.

Vorteilhafterweise kann die Verrechnung der volumetrischen Bilddaten durch Summation der hintereinander angeordneten volumetrischen Bilddaten, wie Voxel oder Vektorelemente, entlang der Durchstrahlrichtung erfolgen.

Dadurch kann beispielsweise jedes Pixel der Projektionsaufnahme durch Summation der Voxel in Durchstrahlrichtung bezüglich der Grauwerte berechnet werden.

Vorteilhafterweise kann die Verrechnung der volumetrischen Bilddaten durch Integration nach der Anwendung einer bestimmten Funktion auf die in Durchstrahlrichtung angeordneten Elemente der volumetrischen Bilddaten erfolgen.

Dies eine weitere Alternative zur Verrechnung der volumetrischen Bilddaten und erlaubt eine unterschiedliche Gewichtung der Elemente nach einer bestimmten Funktion. Diese Funktion kann so gestaltet sein, dass bestimmte Bereiche ausgeblendet beziehungsweise abgeschwächt werden und für die Diagnose wesentlichen Bereiche stärker gewichtet werden.

Vorteilhafterweise kann die Festlegung des Teilvolumens manuell oder computergestützt automatisch erfolgen.

Das Teilvolumen kann manuell durch einen Benutzer unter Verwendung von Eingabemitteln bestimmt werden. Die Festlegung des Teilvolumens kann auch computergestützt automatisch mittels eines Computers und einer Software zur Verarbeitung von Bilddaten unter Verwendung herkömmlicher Bildbearbeitungsverfahren, wie Identifizierung beziehungsweise Segmentierung bestimmter Teilobjekte, wie Zähne oder Kieferknochen, erfolgen.

Vorteilhafterweise kann das Teilvolumen automatisch anhand eines segmentierten Teilobjekts festgelegt werden, wobei die Außenkontur des Teilvolumens entsprechend einer Außenkontur des segmentierten Teilobjekts festgelegt wird.

Zur Segmentierung kann ein herkömmliches Verfahren zur Mustererkennung verwendet werden. Dadurch umfasst ein Volumen nur das Teilobjekt, das diagnostiziert werden soll, so dass das benachbarte Gewebe, wie das Zahnfleisch und der Kieferknochen, unberücksichtigt bleibt und dadurch die Diagnose erleichtert wird.

Vorteilhafterweise kann das Teilvolumen in seiner Außenkontur manuell so festgelegt werden, dass es der Außenkontur eines charakteristischen Teilobjekts entspricht.

Dadurch kann der Benutzer unter Verwendung von Eingabemitteln die Außenkontur des Teilvolumens manuell entsprechend der Außenform des zu diagnostizierenden Teilobjekts, wie eines Zahns oder einer Zahngruppe, festlegen.

Vorteilhafterweise können aus dem 3D-Volumen zunächst die Zähne und/oder der Kieferbogen identifiziert werden. Anschließend wird die Lage und die Orientierung der Zähne und/oder des Kieferbogens im 3D-Volumen bestimmt, um das Teilvolumen und die Durchstrahlrichtung zur Erzeugung der virtuellen Projektionsaufnahme festzulegen.

Die Identifizierung der Zähne und des Kieferbogens aus dem 3D-Volumen kann entweder automatisch computergestützt oder manuell durch einen Benutzer unter Verwendung von Eingabemitteln, wie einer Maus und Tastatur, und unter Verwendung eines Anzeigemittels, wie eines Monitors, erfolgen. Bei der automatischen Identifizierung beziehungsweise Segmentierung werden übliche Verfahren zur Mustererkennung verwendet, bei denen Objekte segmentiert werden, und nach Zusammenhängen zwischen den Objekten gesucht wird. Die Mustererkennung kann die folgenden Schritte umfassen, nämlich die Vorverarbeitung, die Merkmalsgewinnung, die Merkmalsreduktion, und die Klassifizierung der Merkmale. Bei der Vorverarbeitung werden unerwünschte oder irrelevante Bestandteile der Bilddaten entfernt. Bei der Merkmalsgewinnung werden bestimmte Merkmale aus den Bilddaten durch Vergleich mit bekannten Mustern einer Datenbank, wie einer Datenbank von charakteristischen Zähnen, gewonnen. Der automatische Vergleich erfolgt unter Anwendung von Transformationsfunktionen und Skalierung, wobei durch Berechnung einer Varianz zwischen einem Muster aus den Bilddaten und einem erwarteten Muster aus der Datenbank ein Vergleichsfaktor berechnet wird. Bei der Merkmalsreduktion wird geprüft, welche Merkmale für die Klassentrennung relevant sind und welche weggelassen werden können. Insbesondere die gewonnenen Muster von Zähnen und des Kieferbogens sind für dieses Verfahren relevant, wobei die übrigen gewonnenen Merkmale unbeachtet bleiben können. Im letzten Schritt der Klassifizierung werden die wesentlichen erkannten Merkmale, wie Zähne und charakteristische Formgestaltungen des Kieferbogens, in zusammengehörige Klassen, wie Schneidezähne, Backenzähne, Zahnwurzeln und Kieferknochen, unterteilt.

Bei der Merkmalsgewinnung kommen bekannte Verfahren, wie die Rasteranalyse, Cluster-Analyse und Pattern-Matching zur Anwendung.

Vorteilhafterweise kann das Teilvolumen in Form eines Prismas mit einer beliebigen Grundfläche und einer bestimmten Dicke in Durchstrahlrichtung festgelegt werden.

Das Teilvolumen kann computergestützt automatisch anhand bestimmter erkannter Objekte, wie Zähne oder Zähnegruppen, festgelegt werden oder manuell durch den Benutzer eingegeben werden. Zunächst wird eine Grundfläche definiert und anschließend die Dicke in Durchstrahlrichtung bestimmt. Dadurch wird ein geometrisches Prisma gebildet, wobei die Durchstrahlrichtung senkrecht auf der Grundfläche angeordnet sein kann.

Dadurch kann das Teilvolumen so festgelegt werden, dass bestimmte Objekte, wie Zähne oder Zähnegruppen bzw. bestimmte Teile des Kieferknochens, im Teilvolumen angeordnet sind. Durch das Festlegen der Dicke kann das Teilvolumen nur auf das aufzunehmende Objekt begrenzt werden, so dass andere Objekte, wie eine Wange eines Patienten, die vor oder hinter dem aufzunehmenden Objekt in Durchstrahlrichtung angeordnet sind, nicht im Teilvolumen enthalten sind und damit nicht als störende Artefakte in der berechneten Projektionsaufnahme auftauchen.

Vorteilhafterweise kann die virtuelle Projektionsaufnahme in einer Benutzersoftware für die Verwaltung von dentalen Röntgenbildern als eine Intraoralaufnahme abgelegt werden.

Eine virtuelle Projektionsaufnahme in einer Durchstrahlrichtung, die senkrecht zum Verlauf des Kieferbogens lingual bzw. palatinal ausgerichtet ist und dessen Teilvolumen bestimmte Zähne umfasst, entspricht einer Intraoralaufnahme und kann als solche in einer vorhandenen Benutzersoftware abgelegt werden.

Dadurch kann die berechnete Projektionsaufnahme, wie eine herkömmliche Intraoralaufnahme, zur Diagnose verwendet werden.

Vorteilhafterweise kann die virtuelle Projektionsaufnahme mittels einer Anzeigevorrichtung als eine virtuelle Intraoralaufnahme dargestellt werden.

Dadurch kann die virtuelle Projektionsaufnahme mittels einer Anzeigevorrichtung, wie eines Computerbildschirms, in einer vorhandenen Benutzersoftware, wie eine herkömmliche Intraoralaufnahme, dargestellt werden.

Vorteilhafterweise können mehrere Projektionsaufnahmen aus unterschiedlichen Teilvolumina mit unterschiedlichen Durchstrahlrichtungen nach einem vorgegebenen Schema erzeugt werden.

Diese Teilvolumina können beliebig gestaltet sein. Sie können unterschiedliche Dicken mit einer ausgewählten Grundfläche aufweisen oder in ihrer Außenkontur entsprechend einem segmentierten Teilobjekt, wie einem Zahn oder einer Zahngruppe, festgelegt sein. Die erzeugten Projektionsaufnahmen können auch in einer bestimmten Anordnung zueinander mittels der Anzeigevorrichtung dargestellt werden.

Dadurch kann eine Serie von mehreren virtuellen Intraoralaufnahmen vom ganzen Mundraum des Patienten erzeugt werden.

Die Serie der virtuellen Intraoralaufnahmen kann nach einem herkömmlichen Schema erzeugt werden, wie sie im Patent US 5,179,579 in Fig. 5A bis 5S gezeigt ist und in den dazugehörigen Teilen der Beschreibung offenbart ist.

Die einzelnen virtuellen Intraoralaufnahmen der Serie können einzelne oder mehrere Zähne umfassen. Eine virtuelle Intraoralaufnahme von mehreren Zähnen ermöglicht die Diagnose auch von Bereichen zwischen den Zähnen.

Dadurch können Intraoralaufnahmen der einzelnen Zähne oder von Zahngruppen erzeugt werden, um eine verbesserte Diagnose zu ermöglichen. Dadurch wird eine Serie von Intraoralaufnahmen des ganzen Mundraums des Patienten (vollständiger Zahnstatus) erzeugt. Im Gegensatz zu den herkömmlichen Intraoralaufnahmen, die mittels eines Films oder eines Intraoralsensors durchgeführt werden, ist eine exakte Anordnung der virtuellen Teilvolumina möglich, so dass bei einer Serie von Aufnahmen die Teilvolumina lückenlos zueinander angeordnet werden können und damit die Diagnosemöglichkeiten verbessert werden.

Vorteilhafterweise kann jedes Teilvolumen Bilddaten mit volumetrischen Bilddaten eines einzelnen Zahns oder einer Zahngruppe des Kieferbogens umfassen, um separate Projektionsaufnahmen der einzelnen Zähne oder von einzelnen Zahngruppen zu erzeugen.

Dadurch kann eine Serie von Projektionsaufnahmen der einzelnen Zähne beider Kieferbögen erzeugt werden.

Das Teilvolumen kann auch nur Teilbereiche von Zähnen, wie einem halben Zahn umfassen. Das Teilvolumen kann auch einen Zahn und zwei Zahnlücken umfassen.

Vorteilhafterweise kann für jedes Teilvolumen eine Durchstrahlrichtung in palatinaler Richtung zum Gaumen hin beziehungsweise in lingualer Richtung zur Zunge hin festgelegt werden, wobei die einzelnen Teilvolumina aneinander lückenlos anschließen oder sich teilweise überlappen, so dass eine Serie von Projektionsaufnahmen des gesamten Kieferbogens erzeugt wird.

Dadurch entspricht die Durchstrahlrichtung der erzeugten Projektionsaufnahmen der üblichen Orientierung von intraoralen Aufnahmen, so dass die erzeugte Serie, wie eine Serie von herkömmlichen intraoralen Aufnahmen, zur Diagnose verwendet werden kann.

Vorteilhafterweise kann für jedes Teilvolumen eine Durchstrahlrichtung in okklusaler Richtung entlang einer Zahnachse festgelegt werden, wobei die einzelnen Teilvolumina aneinander lückenlos anschließen oder sich teilweise überlappen, so dass eine Serie von okklusalen Projektionsaufnahmen des gesamten Kieferbogens erzeugt wird.

Dadurch wird die Diagnose aus okklusaler Richtung der Zähne des gesamten Kieferbogens ermöglicht.

Vorteilhafterweise kann eine automatische Anordnung der aus dem 3D-Volumen erzeugten virtuellen Projektionsaufnahmen als eine vollständige Erfassung der in der Mundhöhle angeordneten Zähne erfolgen.

Dadurch kann eine Serie von Intraoralaufnahmen (vollständiger Zahnstatus; full mouth series) zur vollständigen Erfassung der Mundhöhle des Patienten erzeugt werden.

Vorteilhafterweise kann die virtuelle Projektionsaufnahme einer mit einer intraoralen Aufnahmevorrichtung bestehend aus einem Röntgenstrahler und einem Bildempfänger, wie einem Intraoralsensor, erzeugten Intraoralaufnahme entsprechen.

Bei einer Durchstrahlrichtung senkrecht zur Ausrichtung des Kieferbogens entspricht die Projektionsaufnahme einer herkömmlichen Intraoralaufnahme eines Abschnitts des Kieferbogens. Die virtuelle Projektionsaufnahme kann auch mit der Durchstrahlrichtung entlang des Kieferbogens für einzelne Zähne erzeugt werden. Solche Projektionsaufnahmen können für die Diagnose bestimmter Befunde im Zwischenraum der Zähne an den approximalen Flächen vorteilhaft sein.

Vorteilhafterweise kann das 3D-Volumen mittels der digitalen Volumentomographie (DVT), mittels der Computertomographie (CT), mittels eines dreidimensionalen Ultraschallverfahrens oder mittels Magnetresonanztomographie (MRT) aufgenommen sein.

Dadurch wird ein 3D-Volumen mit einer hohen Auflösung bei hoher Aufnahmequalität erzeugt.

Vorteilhafterweise können in die Erzeugung der virtuellen Projektionsaufnahme für das festgelegte Teilvolumen bestimmte physikalische Bedingungen einer herkömmlichen Intraoralaufnahme, wie Streuung, Strahlereigenschaften, Röntgenspektrum oder die relative Anordnung des Detektors zum Strahler, einfließen.

Diese Faktoren können beispielsweise, computergestützt simuliert werden oder aus einer Datenbank mit Faktoren für unterschiedliche Anordnungen unter Berücksichtigung unterschiedlicher physikalischer Bedingungen abgerufen werden.

Dadurch wird eine herkömmliche Intraoralaufnahme bezüglich der physikalischen Bedingungen nachgebildet, um eine Diagnose auf herkömmliche Art und Weise zu ermöglichen.

Vorteilhafterweise können bei der Festlegung des Teilvolumens bestimmte störende Teilobjekte, wie beispielsweise Füllungen oder Kieferknochen, aus dem Teilvolumen herausgeschnitten werden oder mit einer geringeren Gewichtung bei der Verrechnung der volumetrischen Bilddaten berücksichtigt werden, um die Untersuchung der vom Teilvolumen umfassten Teilobjekte, wie Zähnen, beispielsweise auf Sekundärkaries hin zu verbessern.

Dadurch kann insbesondere Sekundärkaries, der meist zwischen der Füllung und der Zahnsubstanz angeordnet ist, besser diagnostiziert werden.

Vorteilhafterweise können bei der Erzeugung der virtuellen Projektionsaufnahme für das festgelegte Teilvolumen bestimmte Systemcharakteristika einer herkömmlichen Intraoralaufnahme, wie eine Detektoreffizienz, eine Detektorkennlinie beziehungsweise mehrere Detektorkennlinien und/oder eine Detektorempfindlichkeit bei unterschiedlichen Detektoren, simuliert werden, wobei die Art des Detektors, wie ein Speicherfoliensystem, Zahnfilme unterschiedlicher Empfindlichkeit oder ein digitaler Intraoralsensor, virtuell ausgewählt werden können.

Diese Systemcharakteristika können auch aus einer Datenbank für unterschiedliche Bilddaten abgerufen werden.

Dadurch können Systemcharakteristika bestimmter Detektorarten simuliert werden, um eine herkömmliche Intraoralaufnahme mit dieser Detektorart möglichst genau nachzuahmen. Die Auswahl der Detektorart kann virtuell durch den Benutzer unter Verwendung von Eingabemitteln erfolgen.

### Kurzbeschreibung der Zeichnungen

Ausführungsbeispiele der Erfindung sind in der Zeichnung dargestellt. Es zeigt die
- Fig. 1: eine Skizze eines 3D-Volumens mit ausgewählten Teilvolumina;
- Fig. 2: eine Skizze einer erzeugten Projektionsaufnahme aus einem Teilvolumen;
- Fig. 3: eine Skizze einer Anordnung von mehreren Projektionsaufnahmen des gesamten Mundraums eines Patienten;
- Fig. 4A: eine Skizze eines Teilvolumens mit parallelen Strahlen;
- Fig. 4B: eine Skizze eines alternativen Teilvolumens in Form eines kegelförmigen Fächers;
- Fig. 4C: eine Skizze eines alternativen Teilvolumens mit einer trapezförmigen Grundfläche;
- Fig. 5: eine Skizze eines Schemas mit mehreren ausgewählten Teilvolumina;
- Fig. 6: eine Skizze eines Ausschnitts des 3D-Volumens, das einen einzelnen Backenzahn umfasst;
- Fig. 7: eine Skizze eines alternativen Ausschnitts, das eine Füllung zwischen zwei Backenzähnen umfasst.

### Ausführungsbeispiele

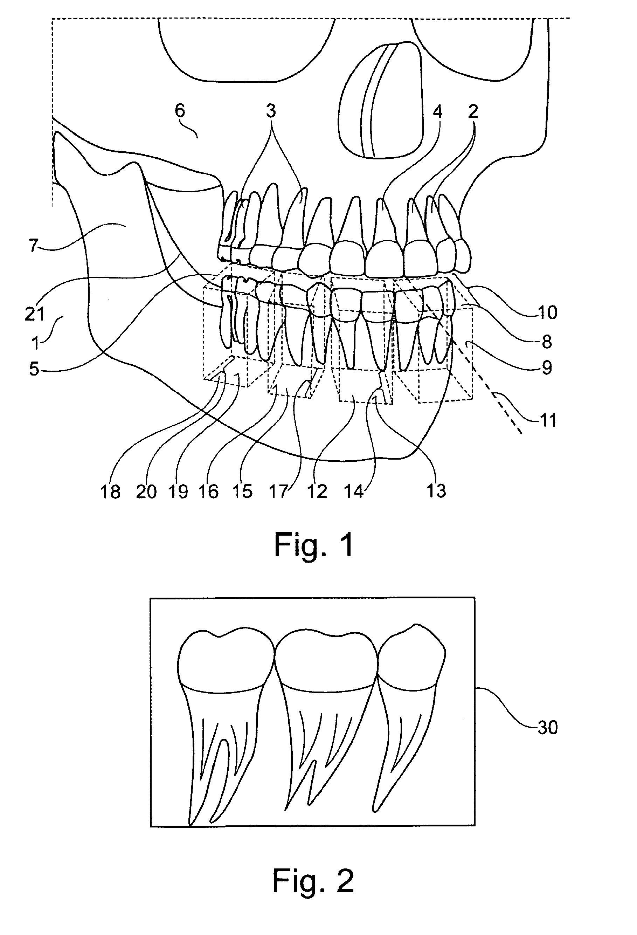

Die Fig. 1 zeigt eine Skizze eines 3D-Volumens 1, das mittels der digitalen Volumentomografie, abgekürzt DVT, oder mittels der Computertomografie, abgekürzt CT, aufgenommen wurde. Das 3D-Volumen 1 besteht aus Voxeln mit bestimmten Röntgenabsorptionswerten. Das 3D-Volumen umfasst mehrere Strukturen, wie die vorderen Schneidezähne 2, die seitlichen Backenzähne 3 und den oberen Kieferbogen 4, den unteren Kieferbogen 5, den oberen Kieferknochen 6 sowie den unteren Kieferknochen 7. Das 3D-Volumen 1 ist in einer Benutzersoftware auf einem nicht gezeigten Computerbildschirm dargestellt. Das 3D-Volumen lässt sich beliebig verschieben und drehen, um die Blickrichtung der Anzeige zu verändern. Im ersten Schritt des Verfahrens wird ein virtuelles Teilvolumen 8 mit einer bestimmten Grundfläche 9 und einer bestimmten Dicke 10 in einer Durchstrahlrichtung 11 festgelegt. Die Festlegung des Teilvolumens kann automatisch computergestützt oder manuell durch einen Benutzer festgelegt werden. Bei der automatischen Festlegung werden mittels eines herkömmlichen Verfahrens zur Mustererkennung die einzelnen Zähne erkannt und klassifiziert, wobei das Teilvolumen so festgelegt wird, dass bestimmte Bereiche des Kieferbogens von diesem Teilvolumen umfasst sind. Bei der manuellen Festlegung kann der Benutzer zunächst mittels Eingabemitteln die Grundfläche 9 definieren und anschließend die Dicke des Teilvolumens bestimmen. Das festgelegte Teilvolumen 8 wird in der Benutzersoftware besonders markiert in Relation zum 3D-Volumen 1 dargestellt. Das zweite Teilvolumen 12 mit der Grundfläche 13 und der Dicke 14, das dritte Teilvolumen 15 mit der Grundfläche 16 und der Dicke 17 sowie das vierte Teilvolumen 18 mit der Grundfläche 19 und der Dicke 20 werden entsprechend festgelegt. Bei der Festlegung der Teilvolumina 8, 12, 15 und 18 ist die Dicke 10, 14, 17, 20 in eine Durchstrahlrichtung ausgerichtet, die senkrecht zum Verlauf 21 der unteren Zahnreihe in palatinaler Richtung zum Gaumen hin bzw. in lingualer Richtung zur Zunge hin angeordnet ist. Die einzelnen Teilvolumina 8, 12, 15 und 18 schließen sich lückenlos aneinander an. Im zweiten Schritt des erfinderischen Verfahrens wird computergestützt unter Verwendung eines Algorithmus aus dem ausgewählten Teilvolumen durch Summation der Röntgenabsorptionswerte in der Durchstrahlrichtung eine zweidimensionale virtuelle Projektionsaufnahme erzeugt, die einer herkömmlichen Intraoralaufnahme des jeweiligen Bereichs des Kieferbogens innerhalb des Teilvolumens entspricht. Die Form der Teilvolumina 8, 12, 15 und 18 entspricht einem Prisma mit einer Grundfläche 9, 13, 16, 19 und der Dicke 10, 14, 17 und 20. Die erzeugte Projektionsaufnahme wird als eine virtuelle Intraoralaufnahme in einer Datenbank einer herkömmlichen Benutzersoftware abgelegt.

Die Fig. 2 zeigt eine Skizze einer erzeugten Projektionsaufnahme 30 aus dem vierten Teilvolumen 18 der hinteren Backenzähne des unteren Kieferbogens 5. Die virtuelle Projektionsaufnahme 30 entspricht einer herkömmlichen Intraoralaufnahme und wird in der Benutzersoftware mittels eines Computerbildschirms angezeigt und entsprechend in einer dafür vorgesehenen Datenbank abgelegt.

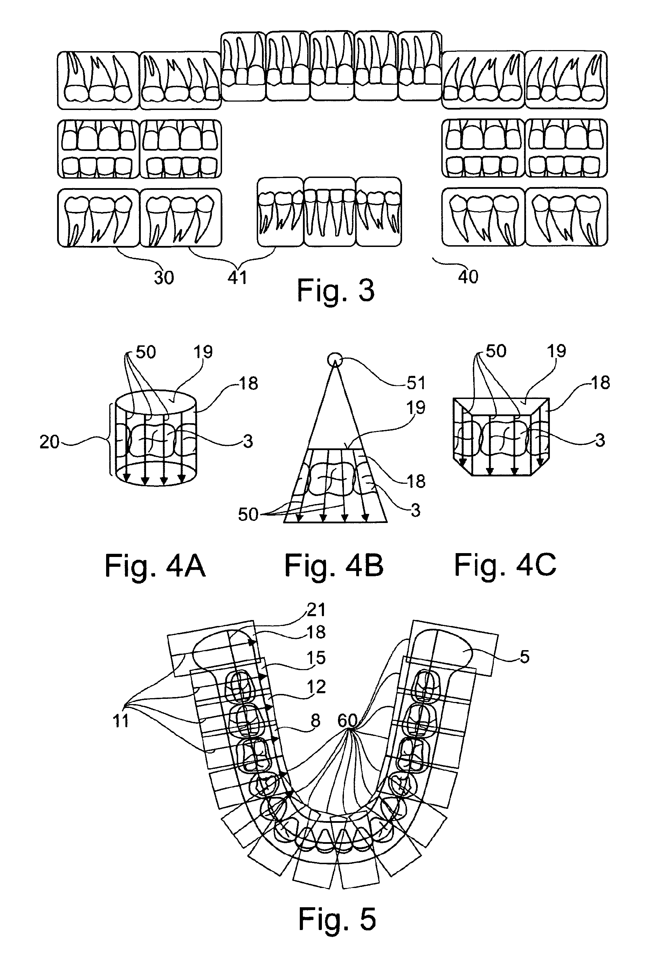

Die Fig. 3 zeigt eine Skizze einer Anordnung 40 von mehreren Projektionsaufnahmen des gesamten Mundraums des Patienten vom unteren Kieferbogen 5 und vom oberen Kieferbogen 6 aus Fig. 1. Unten links ist die erzeugte Projektionsaufnahme 30 aus Fig. 2 zu sehen. Die übrigen Projektionsaufnahmen 41 wurden entsprechend aus den weiteren Teilvolumina 8, 12, 15, die in Fig. 1 dargestellt sind, sowie aus weiteren Teilvolumina des oberen Kieferbogens 6 erzeugt, die in Fig. 1 nicht dargestellt sind. Die Teilvolumina zur Erzeugung der Projektionsaufnahmen 30, 41 aus Fig. 3 sind teilweise überlappend. Die in Fig. 3 gezeigte Anordnung 40 von erzeugten Intraoralaufnahmen 30, 41 wurden nach einem vorgegebenen Schema erzeugt, nämlich nach dem so genannten "full mouth series", auf Deutsch "vollständiger Zahnstatus". Die Projektionsaufnahmen 30, 41 können auch nach einem anderen beliebigen Schema erzeugt werden. Die Erzeugung von Projektionsaufnahmen in Richtung des Verlaufs 21 des unteren Kieferbogens bzw. des oberen Kieferbogens 6 ist ebenfalls möglich, so dass die Bereiche zwischen den Zähnen auf einer solchen Projektionsaufnahme abgebildet werden.

Die Fig. 4A zeigt ein Teilvolumen 18, das einen Backenzahn 3 vollständig und zwei Nachbarzähne zum Teil umfasst, wobei die Grundfläche 19 in Form eines Kreises ausgebildet ist und die Dicke 20 so festgelegt ist, dass die Backenzähne 3 vollständig vom Teilvolumen 18 erfasst sind. Bei der Erzeugung der Projektionsaufnahme aus dem Teilvolumen 18 erfolgt eine Summation entlang der virtuellen Durchstrahlrichtung, die durch virtuelle parallele Strahlen 50 dargestellt ist.

Die Fig. 4B zeigt das Teilvolumen 18, wie in Fig. 4A, mit dem Unterschied, dass die Strahlen 50 in Form eines kegelförmigen Fächers ausgehend von einer virtuellen Röntgenquelle 51 angeordnet sind, wobei die festgelegte Grundfläche 19 ein Rechteck ist.

Die Fig. 4C zeigt eine weitere alternative Ausführungsform des Teilvolumens 18 mit einer trapezförmigen Grundfläche 19, wobei die Durchstrahlrichtung durch parallele Strahlen 50 dargestellt ist.

Die Fig. 5 zeigt ein Schema eines unteren Kieferbogens 5 aus der Sicht von oben mit den ausgewählten Teilvolumina 18, 15, 12, 8 aus Fig. 1 und weiterer Teilvolumina 60, die in die Durchstrahlrichtung 11 angeordnet sind, die senkrecht zum Verlauf 21 des Unterkieferbogens 5 ausgerichtet ist. Bei der Erzeugung der einzelnen Projektionsaufnahmen erfolgt die Summation wie in Fig. 4A oder Fig. 4C dargestellt entlang der parallelen virtuellen Strahlen. Im Unterschied zu Fig. 1 sind die einzelnen Teilvolumina 18, 15, 12, 8 überlappend ausgewählt, so dass Teile des Kieferbogens 5 sowohl von einem Teilvolumen als auch von seinem benachbarten Teilvolumen umfasst sind und damit in den aus diesen benachbarten Teilvolumina erzeugten Projektionsaufnahmen derselbe Teil des Kieferbogens 5 zu sehen ist.

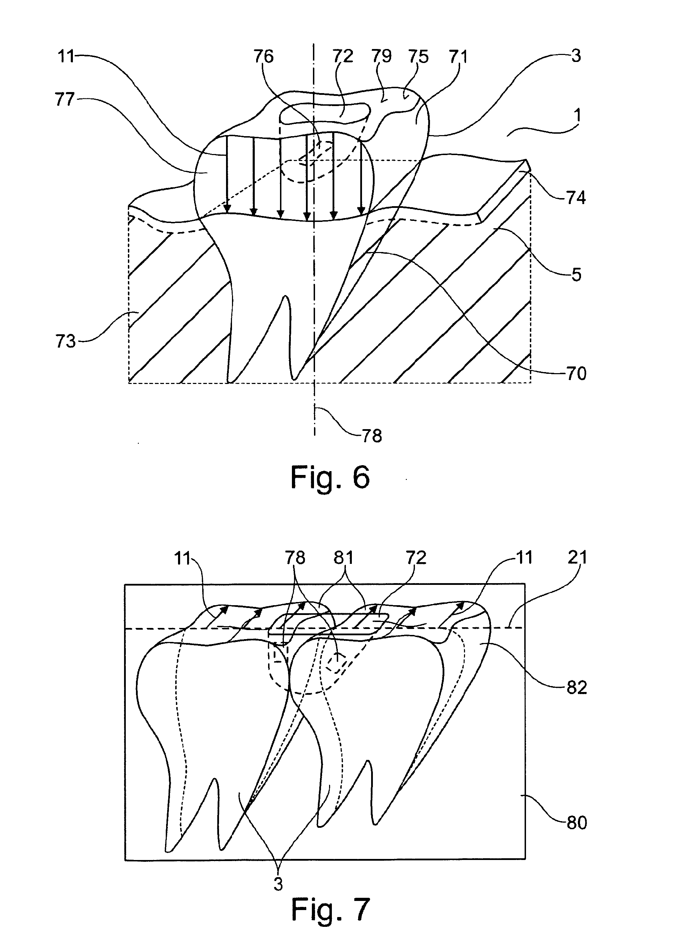

Die Fig. 6 zeigt ein Schema eines Ausschnitts des 3D-Volumens 1, das einen einzelnen Backenzahn 3 des unteren Kieferbogens 5 umfasst. Der Backenzahn 3 besteht aus einer Zahnwurzel 70 und eine Zahnkrone 71, die mit einer Füllung 72 versehen ist. Die Zahnwurzel 70 ist im Kieferknochen 73 angeordnet. Auf dem Kieferknochen 73 liegt die Gingiva 74 auf. Auf der Rückseite 75 der Füllung 72 ist eine Sekundärkaries 76 entstanden, die zu diagnostizieren ist. Ein Teilvolumen 77 ist durch den Benutzer oder computergestützt automatisch festgelegt, wobei das Teilvolumen 77 die Zahnkrone 71 umfasst und das störende Teilobjekt, nämlich die Füllung 72, virtuell herausgeschnitten ist. Die übrigen für die Diagnose unwichtigen Teilobjekte, wie die Zahnwurzel 70, die Gingiva 74, der Kieferknochen 73, wurden ebenfalls bei der Festlegung des Teilvolumens 77 weggelassen. Die virtuelle Durchstrahlrichtung 11 des Volumens 77 verläuft entlang einer Zahnachse 78 in okklusaler Richtung senkrecht zur Okklusalfläche 79. Dadurch dass die Füllung 72 virtuell herausgeschnitten wurde lässt sich anhand der Projektionsaufnahme, die aus dem Teilvolumen 77 in Durchstrahlrichtung 11 erzeugt wird, die Sekundärkaries 76 besser diagnostizieren. Das Ausscheiden der Füllung kann auch mittels einer bestimmten Funktion erfolgen. Eine solche Funktion so gestaltet, dass die für eine Auswertung unwesentliche Bereiche, wie eine Füllung, ausblendet beziehungsweise abschwächt und für eine Auswertung wesentliche Bereiche, wie mit Karies befallen Bereiche, verstärkt dargestellt werden.

Die Fig. 7 zeigt ein Schema eines Ausschnitts des 3D-Volumens 1 wie in Fig. 6 mit dem Unterschied, dass die Füllung 72 zwischen zwei Backenzähnen 3 angeordnet ist und dass die Durchstrahlrichtung 11 in lingualer Richtung senkrecht zum Verlauf 21 des Kieferbogens 5 ausgerichtet ist. In Fig. 7 ist eine Schnittebene 80 dargestellt, die mittig die beiden Backenzähne 3 teilt. Das Teilvolumen 81 ist durch Schnittebene 80 und die Außenkontur 82 der hinteren Seite der Backenzähne 3 gebildet. Dadurch wird die Diagnose von Sekundärkaries 78 auf der Rückseite der Füllung 72 anhand der aus dem Teilvolumen 81 in Durchstahlrichtung 11 erzeugten Projektionsaufnahme verbessert.

### Bezugszeichenliste

- 1: 3D-Volumen
- 2: vordere Schneidezähne
- 3: seitliche Backenzähne
- 4: oberer Kieferbogen
- 5: unterer Kieferbogen
- 6: oberer Kieferknochen
- 7: unterer Kieferknochen
- 8: virtuelles Teilvolumen
- 9: Grundfläche
- 10: Dicke
- 11: Durchstrahlrichtung
- 12: zweites Teilvolumen
- 13: Grundfläche
- 14: Dicke
- 15: drittes Teilvolumen
- 16: Grundfläche
- 17: Dicke
- 18: viertes Teilvolumen
- 19: Grundfläche
- 20: Dicke
- 21: Verlauf
- 30: Projektionsaufnahme
- 40: Anordnung
- 41: Projektionsaufnahme
- 50: Strahlen
- 60: weitere Teilvolumina

## Patentansprüche

1. Verfahren zur Erstellung einer virtuellen dentalen Aufnahme (30, 41) aus einem 3D-Volumen (1) mit volumetrischen Bilddaten, wobei aus dem 3D-Volumen (1) ein Teilvolumen (8, 12, 15, 18) festgelegt wird und wobei für dieses Teilvolumen (8, 12, 15, 18) eine virtuelle Projektionsaufnahme (30, 41) aus einer bestimmten Durchstrahlrichtung (11) durch computergestützte Verrechnung der volumetrischen Bilddaten in dieser Durchstrahlrichtung (11) erzeugt wird, wobei bei der Festlegung des Teilvolumens (77) bestimmte störende Teilobjekte aus dem Teilvolumen (77) unter Anwendung eines Segmentierungsverfahrens herausgeschnitten werden oder mit einer geringeren Gewichtung bei der Verrechnung der volumetrischen Bilddaten berücksichtigt werden, um die Untersuchung der vom Teilvolumen (77) umfassten Teilobjekte zu verbessern, **dadurch gekennzeichnet, dass** mehrere Projektionsaufnahmen (30, 41) aus unterschiedlichen Teilvolumina (8, 12, 15, 18) mit unterschiedlichen Durchstrahlrichtungen (11) nach einem vorgegebenen Schema erzeugt werden und dass entweder für jedes Teilvolumen (8, 12, 15, 18) eine Durchstrahlrichtung (11) in palatinaler Richtung zum Gaumen hin beziehungsweise in lingualer Richtung zur Zunge hin festgelegt wird oder dass für jedes Teilvolumen eine Durchstrahlrichtung (11) in okklusaler Richtung entlang einer Zahnachse (78) festgelegt wird, wobei die einzelnen Teilvolumina (8, 12, 15, 18) aneinander lückenlos anschließen oder sich teilweise überlappen, so dass eine Serie von okklusalen Projektionsaufnahmen des gesamten Kieferbogens (6, 7) erzeugt wird.

2. Verfahren nach Anspruch 1, **dadurch gekennzeichnet, dass** das 3D-Volumen (1) mit volumetrischen Bilddaten eine dreidimensionale Röntgenaufnahme (1) mit Röntgenabsorptionswerten ist.

3. Verfahren nach Anspruch 1 oder 2, **dadurch gekennzeichnet, dass** die Verrechnung der volumetrischen Bilddaten durch Summation der hintereinander angeordneten Elemente der volumetrischen Bilddaten entlang der Durchstrahlrichtung (11) erfolgt.

4. Verfahren nach einem der Ansprüche 1 bis 3, **dadurch gekennzeichnet, dass** vor der Verrechnung der volumetrischen Bilddaten eine bestimmte Funktion auf die volumetrischen Bilddaten in Durchstrahlrichtung angewendet wird, wobei diese Funktion so gestaltet ist, dass bestimmte für die Auswertung wesentlichen Bereiche hervorgehoben werden und andere für die Auswertung unwesentlichen Bereiche weniger gewichtet werden.

5. Verfahren nach einem der Ansprüche 1 bis 4, **dadurch gekennzeichnet, dass** die Festlegung des Teilvolumens (8, 12, 15, 18) computergestützt automatisch erfolgt.

6. Verfahren nach einem der Ansprüche 1 bis 5, **dadurch gekennzeichnet, dass** das Teilvolumen automatisch anhand eines segmentierten Teilobjekts (2, 3) festgelegt wird, wobei die Außenkontur des Teilvolumens entsprechend einer Außenkontur des segmentierten Teilobjekts festgelegt wird.

7. Verfahren nach einem der Ansprüche 1 bis 5, **dadurch gekennzeichnet, dass** das Teilvolumen manuell in seiner Außenkontur so festgelegt wird, dass es der Außenkontur eines charakteristischen Teilobjekts entspricht.

8. Verfahren nach einem der Ansprüche 1 bis 7, **dadurch gekennzeichnet, dass** aus dem 3D-Volumen (1) zunächst die Zähne (2, 3) und/oder der Kieferbogen (6, 7) identifiziert werden, wobei die Lage und die Orientierung der Zähne (2, 3) und/oder des Kieferbogens (6, 7) im 3D-Volumen (1) bestimmt wird, um das Teilvolumen (8, 12, 15, 18) und die Durchstrahlrichtung (11) zur Erzeugung der virtuellen Projektionsaufnahme (30, 41) festzulegen.

9. Verfahren nach einem der Ansprüche 1 bis 8, **dadurch gekennzeichnet, dass** das Teilvolumen (8, 12, 15, 18) mit einer beliebig festgelegten Grundfläche (9, 13, 16, 19) und einer bestimmten Dicke (10, 13, 17, 19) in Durchstrahlrichtung (11) festgelegt wird.

10. Verfahren nach einem der Ansprüche 1 bis 9, **dadurch gekennzeichnet, dass** die virtuelle Projektionsaufnahme (30, 41) in einer Benutzersoftware für die Verwaltung von dentalen Röntgenbildern als eine Intraoralaufnahme abgelegt wird.

11. Verfahren nach einem der Ansprüche 1 bis 10, **dadurch gekennzeichnet, dass** die virtuelle Projektionsaufnahme (30, 41) mittels einer Anzeigevorrichtung als eine Intraoralaufnahme dargestellt wird.

12. Verfahren nach Anspruch 11, **dadurch gekennzeichnet, dass** jedes Teilvolumen (8, 12, 15, 18) Bilddaten mit volumetrischen Bilddaten eines einzelnen Zahns oder einer Zahngruppe des Kieferbogens (6, 7) umfasst, um separate Projektionsaufnahmen (30, 41) der einzelnen Zähne (2, 3) oder von einzelnen Zahngruppen zu erzeugen.

13. Verfahren nach einem der Ansprüche 1 bis 12, **dadurch gekennzeichnet, dass** eine automatische Anordnung der aus dem 3D-Volumen (1) erzeugten virtuellen Projektionsaufnahmen (30, 41) als eine vollständige Erfassung der in der Mundhöhle angeordneten Zähne (2, 3) erfolgt.

14. Verfahren nach einem der Ansprüche 1 bis 13, **dadurch gekennzeichnet, dass** die virtuelle Projektionsaufnahme (30, 41) einer herkömmlichen mit einer intraoralen Aufnahmevorrichtung bestehend aus einem Röntgenstrahler und einem Bildempfänger, wie einem Intraoralsensor, erzeugten Intraoralaufnahme entspricht.

15. Verfahren nach einem der Ansprüche 1 bis 14, **dadurch gekennzeichnet, dass** das 3D-Volumen (1) mittels der digitalen Volumentomographie (DVT), mittels der Computertomographie (CT), mittels eines dreidimensionalen Ultraschallverfahrens oder mittels Magnetresonanztomographie (MRT) aufgenommen ist.

## Claims

1. A method for creating a virtual dental image (30, 41) from a 3D volume (1) having volumetric image data, a sub-volume (8, 12, 15, 18) being defined from the 3D volume (1), wherein a virtual projection image (30, 41) is generated for this sub-volume (8, 12, 15, 18) from a specific irradiation direction (11) by computer-aided computation of the volumetric image data in this irradiation direction (11) and, in the determination of the sub-volume (77), specific disturbing sub-objects from the sub-volume (77) are excised using a segmentation method, or these sub-objects are taken into consideration with a lower weighting in the computing of the volumetric image data in order to improve the examination of the sub-objects included in the sub-volume (77), **characterized in that** a plurality of projection images (30, 41) are created from different sub-volumes (8, 12, 15, 18) having different irradiation directions (11) according to a specified schema, and that either an irradiation direction (11) is defined in the palatinal direction towards the gum or in the lingual direction towards the tongue for each sub-volume (8, 12, 15, 18), or an irradiation direction (11) is defined in the occlusal direction along an axis of the tooth (78) for each sub-volume, the individual sub-volumes (8, 12, 15, 18) adjoining each other with no gaps or partially overlapping, so that a series of occlusal projection images over the entire dental arch (6, 7) is created.

2. The method as recited in claim 1, **characterized in that** the 3D volume (1) having volumetric image data is a three-dimensional X-ray image (1) including X-ray absorption values.

3. The method as recited in claim 1 or claim 2, **characterized in that** the computation of the volumetric image data takes place through summation of the elements of the volumetric image data that are disposed one after the other along the irradiation direction (11).

4. The method as recited in one of claims 1 through 3, **characterized in that** prior to computation of the volumetric image data, a specific function is applied to the volumetric image data in the irradiation direction, this function being configured such that specific regions that are essential for the evaluation are highlighted, and other regions that are not essential for the evaluation are weighted to a lesser degree.

5. The method as recited in one of claims 1 through 4, **characterized in that** the determination of the sub-volume (8, 12, 15, 18) takes place automatically with the aid of a computer.

6. The method as recited in one of claims 1 through 5, **characterized in that** the sub-volume is automatically defined with reference to a segmented sub-object (2, 3), the outer contour of the sub-volume being defined so as to correspond to an outer contour of the segmented sub-object.

7. The method as recited in one of claims 1 through 5, **characterized in that** the sub-volume is manually defined with regard to its outer contour such that it corresponds to the outer contour of a characteristic sub-object.

8. The method as recited in one of claims 1 through 7, **characterized in that** first the teeth (2, 3) and/or the dental arch (6, 7) are identified in the 3D volume (1), the position and the orientation of the teeth (2, 3) and/or of the dental arch (6, 7) in the 3D-volume (1) being determined in order to define the sub-volume (8, 12, 15, 18) and the irradiation direction (11) for the creation of the virtual projection image (30, 41).

9. The method as recited in one of claims 1 through 8, **characterized in that** the sub-volume (8, 12, 15, 18) is defined with an arbitrarily determined basal surface (9, 13, 16, 19) and a specific thickness (10, 13, 17, 19) in the irradiation direction (11).

10. The method as recited in one of claims 1 through 9, **characterized in that** the virtual projection image (30, 41) is stored as an intraoral image in user software for the management of dental X-ray images.

11. The method as recited in one of claims 1 through 10, **characterized in that** the virtual projection image (30, 41) is displayed as an intraoral image using a display device.

12. The method as recited in claim 11, **characterized in that** each sub-volume (8, 12, 15, 18) comprises image data having volumetric image data of an individual tooth or of a group of teeth in the dental arch (6, 7) for the purpose of creating separate projection images (30, 41) of the individual teeth (2, 3) or individual groups of teeth.

13. The method as recited in one of claims 1 through 12, **characterized in that** an automatic arrangement of the virtual projection images (30, 41) created from the 3D volume (1) is carried out as a complete detection of the teeth (2, 3) disposed in the oral cavity.

14. The method as recited in one of claims 1 through 13, **characterized in that** the virtual projection image (30, 41) corresponds to a conventional intraoral image produced using an intraoral imaging device consisting of an X-ray generator and an image receiver, such as an intraoral sensor.

15. The method as recited in one of claims 1 through 14, **characterized in that** the 3D volume (1) is imaged using digital volume tomography (DVT), computer tomography (CT), a three-dimensional ultrasound method, or magnetic resonance tomography (MRT).

## Revendications

1. Procédé pour la réalisation d'un cliché dentaire virtuel (30, 41) à partir d'un volume 3D (1) à l'aide de données d'images volumétriques, un volume partiel (8, 12, 15, 18) étant défini à partir du volume 3D (1) et un cliché de projection virtuel (30, 41) étant généré pour ce volume partiel (8, 12, 15, 18) à partir d'une direction d'irradiation déterminée (11) par une transformation par calcul assistée par ordinateur des données d'images volumétriques dans cette direction d'irradiation (11), des objets partiels gênants, déterminés lors de la définition du volume partiel (77), étant découpés du volume partiel (77) en utilisant un procédé de segmentation ou étant pris en considération avec une pondération plus faible lors de la transformation par calcul des données d'images volumétriques, en vue d'améliorer l'examen des objets partiels englobés par le volume partiel (77), **caractérisé en ce qu'**on génère plusieurs clichés de projection (30, 41) à partir de différents volumes partiels (8, 12, 15, 18) avec différentes directions d'irradiation (11) selon un schéma prédéfini et **en ce qu'**on définit pour chaque volume partiel (8, 12, 15, 18) une direction d'irradiation (11) dans la direction palatine vers la gencive ou dans la direction linguale vers la langue ou **en ce qu'**on définit pour chaque volume partiel une direction d'irradiation (11) dans la direction occlusive le long d'un axe dentaire (78), les différents volumes partiels (8, 12, 15, 18) se raccordant les uns aux autres sans espaces libres ou se chevauchant partiellement de manière à générer une série de clichés de projection occlusifs de l'ensemble des mâchoires (6, 7).

2. Procédé selon la revendication 1, **caractérisé en ce que** le volume 3D (1) avec des données d'images volumétriques est un cliché radiographique tridimensionnel (1) avec des valeurs d'absorption de rayons X.

3. Procédé selon la revendication 1 ou 2, **caractérisé en ce que** la transformation par calcul des données d'images volumétriques est réalisée en effectuant la somme des éléments, disposés les uns derrière les autres, des données d'images volumétriques, le long de la direction d'irradiation (11).

4. Procédé selon l'une quelconque des revendications 1 à 3, **caractérisé en ce qu'**on applique, avant la transformation par calcul des données d'images volumétriques, une fonction déterminée sur les données d'images volumétriques dans le sens d'irradiation, cette fonction étant conçue de manière telle que des zones déterminées essentielles pour l'évaluation sont mises en évidence et que d'autres zones, non essentielles pour l'évaluation, présentent une pondération moindre.

5. Procédé selon l'une quelconque des revendications 1 à 4, **caractérisé en ce que** la détermination du volume partiel (8, 12, 15, 18) est réalisée de manière automatique assistée par ordinateur.

6. Procédé selon l'une quelconque des revendications 1 à 5, **caractérisé en ce que** le volume partiel est déterminé automatiquement à l'aide d'un objet partiel segmenté (2, 3), le contour externe du volume partiel étant déterminé conformément à un contour externe de l'objet partiel segmenté.

7. Procédé selon l'une quelconque des revendications 1 à 5, **caractérisé en ce que** le contour externe du volume partiel est déterminé manuellement de manière telle qu'il correspond au contour externe d'un objet partiel caractéristique.

8. Procédé selon l'une quelconque des revendications 1 à 7, **caractérisé en ce qu'**on identifie, à partir du volume 3D (1), d'abord les dents (2, 3) et/ou les mâchoires (6, 7), la position et l'orientation des dents (2, 3) et/ou des mâchoires (6, 7) étant déterminées dans le volume 3D (1) pour définir le volume partiel (8, 12, 15, 18) et la direction d'irradiation (11) pour la génération du cliché de projection virtuel (30, 41).

9. Procédé selon l'une quelconque des revendications 1 à 8, **caractérisé en ce que** le volume partiel (8, 12, 15, 18) est défini avec une surface de base définie de manière quelconque (9, 13, 16, 19) et une épaisseur déterminée (10, 13, 17, 19) dans la direction d'irradiation (11).

10. Procédé selon l'une quelconque des revendications 1 à 9, **caractérisé en ce que** le cliché de projection virtuel (30, 41) est classé dans un programme d'application pour la gestion de radiographies dentaires sous forme d'un cliché intraoral.

11. Procédé selon l'une quelconque des revendications 1 à 10, **caractérisé en ce que** le cliché de projection virtuel (30, 41) est représenté au moyen d'un dispositif d'affichage sous forme d'un cliché intraoral.

12. Procédé selon la revendication 11, **caractérisé en ce que** chaque volume partiel (8, 12, 15, 18) comprend des données d'images avec données d'images volumétriques d'une seule dent ou d'un groupe de dents des mâchoires (6, 7) pour générer des clichés de projection séparés (30, 41) des différentes dents (2, 3) ou des différents groupes de dents.

13. Procédé selon l'une quelconque des revendications 1 à 12, **caractérisée en ce qu'**une disposition automatique des clichés de projection virtuels (30, 41) générés à partir du volume 3D (1) est réalisée sous forme d'une prise de vue globale des dents (2, 3) disposées dans la cavité buccale.

14. Procédé selon l'une quelconque des revendications 1 à 13, **caractérisé en ce que** le cliché de projection virtuel (30, 41) correspond à un cliché intraoral généré de manière classique à l'aide d'un dispositif d'enregistrement intraoral constitué par un émetteur de rayons X et un capteur d'images, tel qu'une sonde intraorale.

15. Procédé selon l'une quelconque des revendications 1 à 14, **caractérisé en ce que** le volume 3D (1) est enregistré au moyen de la tomographie volumétrique numérique (DVT), au moyen de la tomodensitométrie (CT), au moyen d'un procédé tridimensionnel à ultrasons ou au moyen de l'imagerie par résonance magnétique (IRM).
